(19)

Europäisches
Patentamt
European
Patent Office
Office européen
des brevets

(11) **EP 3 805 763 A1**

(12) **EUROPEAN PATENT APPLICATION**
published in accordance with Art. 153(4) EPC

(43) Date of publication:
14.04.2021 Bulletin 2021/15

(21) Application number: 19812013.1

(22) Date of filing: 27.05.2019

(51) Int Cl.:
*G01N 33/74* (2006.01)          *A61F 13/511* (2006.01)
*A61F 13/84* (2006.01)          *G01N 33/36* (2006.01)

(86) International application number:
PCT/JP2019/020888

(87) International publication number:
WO 2019/230647 (05.12.2019 Gazette 2019/49)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 29.05.2018  JP 2018102883
24.05.2019  JP 2019098044

(71) Applicant: Kao Corporation
Chuo-ku,
Tokyo 103-8210 (JP)

(72) Inventors:
• FUKUDA, Yuko
Haga-gun, Tochigi 321-3497 (JP)
• YAMANAKA, Yuki
Tokyo 131-0044 (JP)
• SAKAMOTO, Takashi
Haga-gun, Tochigi 321-3497 (JP)
• TOMITA, Mina
Haga-gun, Tochigi 321-3497 (JP)

(74) Representative: Vossius & Partner
Patentanwälte Rechtsanwälte mbB
Siebertstrasse 3
81675 München (DE)

(54) **METHOD FOR EVALUATING COMFORT EVOKING PERFORMANCE OF SHEET AND COMFORT EVOKING SHEET**

(57)    The present invention provides a method for evaluating comfort evoking performance of sheets, with which whether or not a sheet has an ability to evoke comfort as a result of the sheet being touched, or a level of the ability is evaluated. The method includes the following steps (A) to (D):
(A) bringing a test sheet, which is an evaluation target, into contact with the skin or hairs of an animal and applying a tactile stimulation to the animal;
(B) extracting a biological sample from the animal after the tactile stimulation has been applied;
(C) measuring an amount of oxytocin in the biological sample; and
(D) comparing the amount of oxytocin measured with an amount of oxytocin in a biological sample extracted from the animal under a condition where there is no effect of the tactile stimulation.

Fig. 2

**Description**

Technical Field

The present invention relates to a method for evaluating the comfort evoking performance of sheet, and a comfort evoking sheet.

Background Art

An absorbent article such as a disposable diaper is known that includes a constituent member that contains oxytocin or an aromatic component that induces oxytocin, from the viewpoint of continuously giving comfort to a user. For example, Patent Literature 1 discloses an absorbent article that detachably includes an attaching member to which oxytocin or a component that induces oxytocin is applied. Also, Patent Literature 2 discloses an absorbent article that includes a specific area to which an aromatic substance that releases an aromatic component by a predetermined stress being applied, wherein the aromatic component contains oxytocin or a component that induces oxytocin.

Citation List

Patent Literatures

    Patent Literature 1: WO 2014/092087
    Patent Literature 2: JP 2014-113302A

Non-Patent Literatures

    Non-Patent Literature 1: Lieberwirth and Wang, CURRENT OPINION IN NEUROBIOLOGY, doi: 10.1016/j.conb.2016.05.006, 2016
    Non-Patent Literature 2: Loken et al., NATURE NEUROSCIENCE, Vol. 12, p547-548, 2009
    Non-Patent Literature 3: Rie Hikima, et al., Program of the 35th Meeting of the Japanese Society for Physiological Psychology and Psychophysiology · Preprints, 59, 2017
    Non-Patent Literature 4: Morhenn et al., Altern. Ther. Health. Med., 18, 11-18, 2012
    Non-Patent Literature 5: Andari E., et al., 2010, Proc Natl Acad Sci USA, 107(9):4389-94

Summary of Invention

The present invention relates to a method for evaluating comfort evoking performance of sheets, with which whether or not a sheet has an ability to evoke comfort as a result of the sheet being touched, or a level of the ability is evaluated, the method including the following steps (A) to (D):

    (A) bringing a test sheet, which is an evaluation target, into contact with the skin or hairs of an animal and applying a tactile stimulation to the animal;
    (B) extracting a biological sample from the animal after the tactile stimulation has been applied;
    (C) measuring an amount of oxytocin in the biological sample; and
    (D) comparing the amount of oxytocin measured with an amount of oxytocin in a biological sample extracted from the animal under a condition where there is no effect of the tactile stimulation.

Also, the present invention relates to a method for designing an absorbent article. The designing method includes performing evaluation on a plurality of sheets using the evaluation method described above, and determining a sheet selected based on a result of the evaluation as a constituent member that is brought into contact with skin.

Also, the present invention relates to a method for producing an absorbent article. The production method includes producing an absorbent article designed based on the designing method described above by using the selected sheet.

Also, the present invention relates to a comfort evoking sheet that is determined as having an ability to evoke comfort by being touched, using a method for evaluating a sheet including the following steps (A) to (C), (D), and (E),

(A) bringing a test sheet, which is an evaluation target, into contact with the skin or hairs of an animal and applying a tactile stimulation to the animal;

(B) extracting a biological sample from the animal after the tactile stimulation has been applied;

(C) measuring an amount of oxytocin in the biological sample;

(D) comparing the amount of oxytocin measured with an amount of oxytocin in a biological sample extracted from the animal under a condition where there is no effect of the tactile stimulation; and

(E) determining, based on a rate of change in the amount of oxytocin or a difference in the amount of oxytocin obtained as a result of the comparison in the step (D), that the test sheet has the ability to evoke comfort by being touched.

Also, the present invention relates to an article in which the comfort evoking sheet is used. The article is configured such that either one surface of the sheet is provided so as to be capable of being brought into contact with human skin. The article is any one of clothes, accessories, appliances, bedclothes, covers, and toys.

Also, the present invention relates to an article for babies and infants in which the comfort evoking sheet is used. The article is configured such that either one surface of the sheet is provided so as to be capable of being brought into contact with the skin of a human. The article is any one of clothes, accessories, diapers, bedclothes, and toys.

Brief Description of Drawings

[Fig. 1] Fig. 1 is a schematic diagram showing an example of a mode in which a tactile stimulation is applied in an evaluation method according to the present invention.

[Fig. 2] Fig. 2 is a schematic diagram showing an example of a mode in which a tactile stimulation is applied to an article that includes a test sheet according to the present invention as a constituent member.

[Fig. 3] Fig. 3 is a graph showing the amount of oxytocin before and after a tactile stimulation is applied to sheets produced in Examples 1 and 2 and Comparative Example 1.

Detailed Description of the Invention

Oxytocin is peptide hormone synthesized primarily in the hypothalamus of the brain, and is reported as being involved in forming affection and sociability between living organisms (Non-Patent Literature 1).

However, if oxytocin itself or a substance that induces oxytocin is applied to a constituent member of an absorbent article as in the absorbent articles disclosed in Patent Literatures 1 and 2, it may have an excessive physiological influence on a user such as a wearer who has touched these substances. In addition, it is necessary to be very careful when adjusting the amounts of these substances.

In recent years, there have been reports that various types of tactile stimulation can evoke a comforting feeling of being comfortable. For example, Non-Patent Literature 2 reports that comfort evokes as a result of a tactile stimulation, which is applied by the skin being rubbed with a comfort evoking brush member, being conveyed to the brain through the C fibers. Non-Patent Literature 3 reports that comforting feelings including a feeling of happiness, a feeling of satisfaction, a luxurious feeling, and a feeling of excitement evoke when the face is covered with the palms, with the eyes being closed.

Also, Non-Patent Literature 4 reports that a massage that is one form of the tactile stimulation that evokes comfort increases the amount of oxytocin in a living organism. Also, Non-Patent Literature 5 reports that comfort caused by a sense of touch increases as a result of the amount of oxytocin in a living organism increasing upon transnasally applying oxytocin to the living organism.

As described above, there have been reports on the relationship between tactile stimulation and the amount of oxytocin in living organisms. However, what kind of feel is needed to produce the tactile stimulation that increases the amount of oxytocin in living organisms has not yet been known, and thus further studies are required to identify such a touch.

The inventors of the present invention conducted studies for a sheet that has comfort evoking performance that increases the amount of oxytocin as a result of a tactile stimulation being applied, and found an evaluation method, which it is possible to objectively and quantitatively evaluate comfort evoking performance.

The present invention relates to an evaluation method, with which whether or not a sheet has an ability to evoke comfort by being touched, or a level of the ability is evaluated. The ability to evoke comfort by being touched is also called "comfort evoking performance".

Hereinafter, an evaluation method according to the present invention will be described by way of a preferred mode thereof with reference to the drawings.

According to the present mode, the method includes the following steps (A) to (D):

(A) bringing a test sheet, which is an evaluation target, into contact with the skin or hairs of an animal and applying a tactile stimulation to the animal;
(B) extracting a biological sample from the animal after the tactile stimulation has been applied;
(C) measuring an amount of oxytocin in the biological sample; and
(D) comparing the amount of oxytocin measured with an amount of oxytocin in a biological sample extracted from the animal under a condition where there is no effect of the tactile stimulation.

In the step (A), the animal to which the test sheet is brought into contact may be a mammal, including a human, that produces oxytocin. Examples of the animal other than a human include pets such as a dog, livestock such as a cow and a pig, and other non-human animals such as a chimpanzee, a monkey, a rabbit, a guinea pig, a rat, and a mouse (Yamashita and Kitano, Mol. Phylogenet. Evol., 2013, 2, 520 - 528).

A tactile stimulation is applied to the animal by bringing a test sheet into contact with the skin or hairs of the animal. The area of the animal that is contacted by the test sheet will also be referred to as a "contact area". There is no particular limitation on the contact area as long as it is located on the skin or hairs of the animal. The contact area can be located, for example, on the hands including the fingers, the palm, the back of the hand, the upper arms, the elbows, the lower arms, and the like, the feet including foot fingers, the sole of the foot, and the like, the thighs, the back, the chest, the shoulders, the neck, the head, the hips, and the like. The skin can be classified according to the presence or absence of hair roots into a haired portion in which there are hair roots and a non-haired portion in which there are no hair roots. However, the skin may be either one or both of the haired portion and the non-haired portion. In the present mode, the non-haired portion may be the palm, the sole of the foot, or the like, and mucous membranes are excluded. The term "hair" of the animal includes head hair and body hair.

The tactile stimulation is a stimulation perceived as a result of the test sheet and the contact area being brought into contact with each other. From the viewpoint of more reliably applying the tactile stimulation, it is preferable to bring the test sheet into contact with the contact area while the test sheet is fixed. In this case, one surface of the test sheet is brought into contact with the contact area. In the description of the present application, one surface of the sheet that is brought into contact with the contact area will be also referred to as a "skin-facing surface".

In the step (A), the tactile stimulation may be applied in a stationary state in which the contact area is stationary while it is in contact with the test sheet or in a motion state in which the contact area moves while it is in contact with the test sheet. From the viewpoint of more reliably applying the tactile stimulation, it is preferable to apply the tactile stimulation in the motion state. The tactile stimulation in the motion state may be applied by the contact area rubbing the test sheet in the plane direction, the contact area lightly pressing the test sheet in the thickness direction of the test sheet, or the like.

The contact between the contact area and the test sheet may be performed by the animal itself in a voluntary manner or in a forced manner.

In the step (A), the tactile stimulation may be applied continuously or intermittently. In the case of intermittently applying the tactile stimulation, applying the tactile stimulation and maintaining a resting state are alternately performed such that the contact area is contacted with the test sheet to apply the tactile stimulation and then the contact area is left without being touched.

There is no particular limitation on the time during which the tactile stimulation is applied. However, in the case of intermittently applying the tactile stimulation, the total time during which the contact area and the test sheet are in contact with each other is preferably set as the time during which the tactile stimulation is applied.

In the step (B), a biological sample is extracted from the animal to which the tactile stimulation was applied in the step (A). The timing at which the biological sample is extracted from the animal is after the tactile stimulation has been applied, and is preferably within 60 minutes after the tactile stimulation has been applied. However, the biological sample may be extracted from the animal during a period from while the tactile stimulation is being applied to a time at which the tactile stimulation reaches a predetermined time. Alternatively, the biological sample may be extracted from the animal at the timing when a predetermined period of time passes after the tactile stimulation is applied and the contact area is resting state.

In the step (B), the biological sample to be extracted may be, specifically, blood, urine, saliva, lymph fluid, or the like. Blood plasma, blood serum, or blood cells (red blood cells, white blood cells) may be fractionated from blood using a known method, and any one of these may be used as the biological sample.

The extracted biological sample is preferably frozen and stored using dry ice immediately after extraction.

In the step (C), the amount of oxytocin in the biological sample extracted in the step (B) is measured. There is no particular limitation on the method for measuring the amount of oxytocin, and an immunological method can be used such as a liquid chromatograph (HPLC), a liquid chromatography-mass spectrometer (LC-MS), a liquid chromatography tandem-mass spectrometer (LC-MS/MS), a gas chromatography-mass spectrometer (GC-MS), or an enzyme linked immunosorbent assay (ELISA). The measurement conditions using any of the above methods are known, and thus the amount of oxytocin can be easily quantified according to a conventional method. The ELISA method can be carried out using, for example, Oxytocin ELISA kit (Enzo).

In the step (D), the amount of oxytocin measured in the step (C) is compared with the amount of oxytocin in the biological sample extracted from the animal under a condition where there is no effect of the tactile stimulation, and the presence or absence of comfort evoking performance is checked. The comparison of the amount of oxytocin in the biological sample in the step (D) may be performed by comparing a biological sample to which the tactile stimulation applied has been applied with a biological sample at the time when there is no effect of the tactile stimulation, the biological samples being extracted from animals of the same population. In this case, the expression "the biological sample extracted from the animal under a condition where there is no effect of the tactile stimulation" corresponds to the expression "the biological sample at the time when there is no effect of the tactile stimulation".

In the comparison between the biological samples extracted from animals of the same population, the expression "the time when there is no effect of the tactile stimulation" refers to the time when the amount of oxytocin does not vary due to the tactile stimulation, and it may be, for example, the time when the amount of oxytocin no longer varies due to the tactile stimulation after a predetermined period of time passes after the tactile stimulation has been applied, or the time before the tactile stimulation is applied.

That is, in the case where the biological samples extracted from animals of the same population are compared in the step (D), either one of the following steps (D1) and (D2) is carried out:

(D1) comparing the amount of oxytocin measured with an amount of oxytocin in a biological sample extracted from the animal before the tactile stimulation is applied; and
(D2) comparing the amount of oxytocin measured with an amount of oxytocin in a biological sample extracted from the animal at a time when the amount of oxytocin no longer varies due to the tactile stimulation after the tactile stimulation is applied.

Alternatively, the comparison of the amount of oxytocin between biological samples in the step (D) may be performed by comparing a biological sample obtained from a population to which the tactile stimulation is applied with a biological sample obtained from another population to which the tactile stimulation is not applied. The two populations preferably have the same attributes, with the same factors such as type, age, gender, the experience of childbirth, and the experience of parenting. In the case where biological samples obtained from different populations are compared, the expression "under a condition where there is no effect of the tactile stimulation" corresponds to the expression "another population to which the tactile stimulation is not applied".

In the step (D), in the case where biological samples obtained from different populations are compared, the following step (D3) is carried out:

(D3) comparing the amount of oxytocin measured that is an amount of oxytocin in a biological sample extracted from a population to which the tactile stimulation is applied with an amount of oxytocin in a biological sample extracted from another population to which the tactile stimulation is not applied.

In the steps (D1) to (D3), the amount of oxytocin in the biological sample extracted from the animal under a condition where there is no effect of the tactile stimulation will be referred to as "the amount of oxytocin in the steady state". Also, the amount of oxytocin measured in the step (C) may also be referred to as "the amount of oxytocin at the time of applying the tactile stimulation".

As described above, an increase in the amount of oxytocin can evoke comfort. For this reason, if, in the step (D), the amount of oxytocin at the time of applying the tactile stimulation is larger than the amount of oxytocin in the steady state, it can be determined that the test sheet has comfort evoking performance.

On the other hand, if the amount of oxytocin at the time of applying the tactile stimulation is less than or equal to the amount of oxytocin in the steady state, it can be determined that the test sheet does not have comfort evoking performance. In this way, by comparing the amount of oxytocin at the time of applying the tactile stimulation with the amount of oxytocin in the steady state, the ability to increase the amount of oxytocin can be evaluated objectively and quantitatively. With this configuration, it is possible to find a sheet with comfort evoking performance and evaluate the level of comfort evoking performance of the sheet, and thus development of sheets that can increase the amount of oxytocin by the tactile stimulation can be effectively performed.

The determination as to whether or not the sheet has comfort evoking performance based on a result the comparison of the amount of oxytocin in the step (D) can be made based on, for example, the rate of change (%) in the amount of oxytocin at the time of applying the tactile stimulation with respect to the amount of oxytocin in the steady state, or based on the difference between the amount of oxytocin in the steady state and the amount of oxytocin at the time of applying the tactile stimulation. In the case where the comparison is performed based on the rate of change (%), if the rate of change in the amount of oxytocin at the time of applying the tactile stimulation with respect to the amount of oxytocin in the steady state increases by an amount greater than or equal to a predetermined value, for example, preferably 10% or more, more preferably 30% or more, it is determined that the test sheet has comfort evoking performance. In the case where the biological samples extracted from animals of the same population are compared in the step (D), or in other words, in the case where the step (D1) or (D2) is carried out, if the proportion of the amount of oxytocin at the time of applying the tactile stimulation with respect to the amount of oxytocin in the steady state, or in other words, the rate of change in the amount of oxytocin increases by an amount corresponding to preferably 10% or more, more preferably 30% or more, it is determined that the test sheet has comfort evoking performance. In the case where population comparison is performed in the step (D), or in other words, in the case where the step (D3) is performed, if the amount of oxytocin of the biological sample extracted from the population to which the tactile stimulation has been applied is larger than the amount of oxytocin of the biological sample extracted from the population to which the tactile stimulation is not applied by an amount corresponding to preferably 10% or more, and more preferably 30% or more, it is determined that the test sheet has comfort evoking performance.

Also, in the case where the determination as to whether or not the sheet has comfort evoking performance is made based on the difference between the amount of oxytocin in the steady state and the amount of oxytocin at the time of applying the tactile stimulation, based on the assumption that the amount of oxytocin at the time of applying the tactile stimulation is larger than the amount of oxytocin in the steady state, in the step (D), the difference between the amount of oxytocin in the steady state and the amount of oxytocin at the time of applying the tactile stimulation is obtained, and if the difference is greater than or equal to a predetermined value, it may be determined that the test sheet has comfort evoking performance. Whether or not the difference is greater than or equal to the predetermined value can be determined based on, for example, the ratio of the difference between the amount of oxytocin in the steady state and the amount of oxytocin at the time of applying the tactile stimulation with respect to the amount of oxytocin in the steady state. This ratio will also be referred to simply as "difference ratio". The difference ratio H is represented by the following equation:

$$\text{Difference ratio H (\%)} = [(H2 - H1) / H1] \times 100,$$

where the amount of oxytocin in the steady state is represented by HI, and the amount of oxytocin at the time of applying the tactile stimulation is represented by H2.

In the case where the step (D1) or (D2) is carried out in the step (D), if the difference ratio H is greater than or equal to a predetermined value, for example, takes a value as high as preferably 10% or more, and more preferably 30% or more, it is determined that the test sheet has comfort evoking performance. Also, in the case where the step (D3) is carried out in the step (D), the amount of oxytocin of the biological sample extracted from the population to which the tactile stimulation has been applied is defined as the amount of oxytocin at the time of applying the tactile stimulation H2, and the amount of oxytocin of the biological sample extracted from the population to which the tactile stimulation is not applied is defined as the amount of oxytocin in the steady state HI, and if the difference ratio H takes a value as high as preferably 10% or more, and preferably 30% or more, it is determined that the test sheet has comfort evoking performance.

Also, whether or not the difference between the amount of oxytocin in the steady state and the amount of oxytocin at the time of applying the tactile stimulation is greater than or equal to a predetermined value may be determined based on the absolute value of the difference.

Determining whether or not the sheet has comfort evoking performance described above is performed in the step (E), which will be described later.

In the case where the step (D3) is carried out in the step (D), whether or not there is a significant difference in the amount of oxytocin between two populations in the steady state and at the time of applying the tactile stimulation may be determined using a statistical test method such as t-test, Mann-Whitney U test, Wilcoxon signed-rank test, or Dunnett-test.

From the viewpoint of preventing the possibility of receiving an excessive physiological influence caused by oxytocin or an oxytocin inducing agent, in the evaluation method according to the present mode, it is preferable that the test sheet does not contain oxytocin or an oxytocin inducing agent. With the evaluation method according to the present mode, even if oxytocin or an oxytocin inducing agent is not contained, a sheet that can increase the amount of oxytocin due to contact can be found.

As the oxytocin inducing agent, for example, a substance that contains breast milk as the main component, rose oil, ethyl trimethylcyclo pentenyl butenol, methyl trimethylcyclo pentenyl pentanol, hexahydrohexamethyl cyclopentabenzopyran, and the like are known (Patent Literature 1).

In the step (D), comparison between the amount of oxytocin at the time of applying the tactile stimulation and the amount of oxytocin in the steady state is performed, but from the viewpoint of performing more accurate evaluation of comfort evoking performance, the amount of oxytocin in the steady state is preferably the amount of oxytocin before the tactile stimulation is applied by the test sheet being touched. That is, in the step (D), it is preferable to carry out the step (D1).

The timing at which the biological sample is extracted before the tactile stimulation is applied by the test sheet being touched is preferably during a period from 30 minutes to immediately (0 minutes) before the tactile stimulation is applied, and more preferably during a period from 15 minutes to immediately (0 minutes) before the tactile stimulation is applied. The extraction of the biological sample should be finished before the tactile stimulation is applied. It takes, for example, 5 minutes or more and 15 minutes or less to extract the biological sample.

From the viewpoint of easily applying the tactile stimulation to the animal, in the step (A), it is preferable to apply the tactile stimulation to the animal by moving a haired portion or a non-haired portion on the skin of the animal while it is in contact with the test sheet. That is, the contact area is a haired portion or a non-haired portion of the skin. The haired portion is preferably the back of the hand, the posterior of the forearm, the cheek of the face, and more preferably the back of the hand. The non-haired portion is preferably the palm, the sole of the foot, and more preferably the palm. The term "palm" as used herein encompasses, in addition to the strict definition of "palm" extending from the wrist to the roots of fingers, the palmar side of fingers .

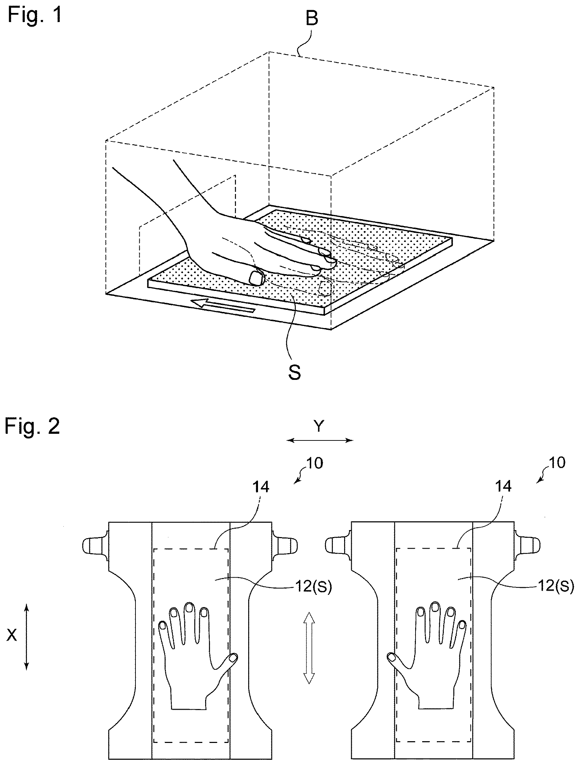

From the viewpoint of reducing the influence of the amount of oxytocin due to a stimulation other than the tactile stimulation, as shown in Fig. 1, the tactile stimulation is preferably applied in a state in which the test sheet cannot be visually seen. In the mode shown in Fig. 1, the tactile stimulation is applied by placing a test sheet S in a blind box B with an opening into which a hand can be inserted, and bringing the hand inserted from the opening into contact with the test sheet S.

Also, from the viewpoint of accurately determining or evaluating the comfort evoking performance of the sheet according to the mode thereof suitable for the use/application of the sheet, the test sheet may be shaped to be suitable for its use/application as shown in Fig. 2 such that the tactile stimulation can be applied while visually recognizing the test sheet. In the mode shown in Fig. 2, the tactile stimulation is applied by incorporating the test sheet S into a diaper as a top sheet 12 of the diaper and bringing a hand into contact with the test sheet S. In the case where the animal is a human, from the viewpoint of more accurately determining or evaluating comfort evoking performance, it is preferable to apply the tactile stimulation after informing that "evaluation of the material of the disposable diaper" is to be performed.

In the mode shown in Fig. 1, the tactile stimulation is applied to a human by bringing the palm of one hand of the human into contact with the test sheet S. However, as in the mode shown in Fig. 2, the tactile stimulation may be applied to a human by bringing the palms of both hands of the human into contact with the test sheets S. In Fig. 2, two diapers 10 are prepared, and the palms of the right and left hands are brought into contact with the top sheets 12 of the diapers 10, respectively.

The diapers 10 shown in Fig. 2 each include a top sheet 12, a back sheet (not shown), and an absorbent member 14 that is provided between the top sheet 12 and the back sheet. The top sheet 12 is provided at a position at which it can come into contact with the skin of the wearer.

From the viewpoint of easily exhibiting the comfort evoking performance of the sheet, it is preferable to apply the tactile stimulation to the animal by moving the contact area while it is in contact with the surface of the test sheet. That is, it is preferable to apply the tactile stimulation in the above-described motion state.

From the viewpoint of more reliably producing the above-described effect, it is preferable to apply the tactile stimulation to a haired portion or a non-haired portion on the skin of the animal in a state of motion. For example, the haired portion on the skin such as the back of a hand or the non-haired portion such as a palm is moved while it is in contact with the surface of the test sheet. In this case, it is preferable to move the haired portion or the non-haired portion in a direction parallel to the surface of the test sheet, and it is more preferable to repeatedly move the haired portion or the non-haired portion back and forth in a direction parallel to the surface of the test sheet and a direction opposite thereto.

There is no particular limitation on the moving speed at which the contact area such as the haired portion or the non-haired portion is moved when applying the tactile stimulation thereto. Also, in the case where the contact area is moved back and forth, there is no particular limitation on the number of repetitions of moving back and forth. However, the number of times of moving back or forth is preferably 10 times or more and 300 times or less, and more preferably 15 times or more and 200 times or less.

In the case where the tactile stimulation is applied to a palm of the animal in a state of motion, for example, the palm of one hand may be brought into contact with the surface of the test sheet as shown in Fig. 1, or the palms of both hands may be brought into contact with the surface of the test sheet.

From the viewpoint of more reliably applying the tactile stimulation to the animal, in the step (A), the length of time during which the tactile stimulation is applied continuously or intermittently to the animal is preferably 30 seconds or more, more preferably 45 seconds or more, and even more preferably 60 seconds or more, and preferably 600 seconds or less, more preferably 450 seconds or less, and even more preferably 300 seconds or less, and preferably 30 seconds or more and 600 seconds or less, more preferably 45 seconds or more and 450 seconds or less, and even more preferably 60 seconds or more and 300 seconds or less.

From the viewpoint of more reliably producing the above-described effect, it is preferable to continuously apply the tactile stimulation to the animal, and the length of time during which the tactile stimulation is continuously applied is preferably 30 seconds or more, more preferably 45 seconds or more, and even more preferably 60 seconds or more, and preferably 300 seconds or less, more preferably 240 seconds or less, and even more preferably 180 seconds or less, and preferably 30 seconds or more and 300 seconds or less, more preferably 45 seconds or more and 240 seconds or less, and even more preferably 60 seconds or more and 180 seconds or less.

From the viewpoint of avoiding physical exhaustion of the contact area and the vicinity thereof at the time of applying the tactile stimulation and mental exhaustion caused by limiting the movement of the animal so as to apply the tactile stimulation, in the step (A), it is preferable to intermittently apply the tactile stimulation. In this case, in the step (A), a cycle of applying the tactile stimulation and maintaining a resting state is repeated. The length of time during which the tactile stimulation is applied is preferably 15 seconds or more and 180 seconds or less, and more preferably 30 seconds or more and 120 seconds or less. Also, the length of time during which the resting state is maintained is preferably 15 seconds or more and 180 seconds or less, and more preferably 30 seconds or more and 120 seconds or less. The cycle of applying the tactile stimulation and maintaining the resting state is repeated preferably 3 times or more and 15 times or less, and more preferably 5 times or more and 10 times or less.

From the viewpoint of accurately monitoring the effect of increasing the amount of oxytocin after the tactile stimulation has been applied, in the step (B), the biological sample is extracted from the animal preferably within 50 minutes, and more preferably within 40 minutes after the tactile stimulation has been applied. Also, the biological sample is extracted from the animal preferably within 65 minutes, more preferably within 55 minutes, and even more preferably within 45 minutes after the tactile stimulation has been applied, when the time is measured from when the application of the tactile stimulation starts by taking into consideration the length of time during which the tactile stimulation is applied continuously or intermittently. In the case of intermittently applying the tactile stimulation, the cycle of applying the tactile stimulation and maintaining the resting state is repeated during a period from when the application of the tactile stimulation starts to when the biological sample is extracted.

There is no particular limitation on the biological sample extracted in the step (B), but, from the viewpoint if easily performing the extraction operation, the biological sample is preferably blood, blood serum, blood plasma, urine, or saliva, and more preferably saliva.

In the case where blood, blood serum, or blood plasma is extracted as the biological sample, the method of taking blood and the method of separating blood serum or blood plasma from blood can be performed using known methods.

In the case where saliva is extracted as the biological sample, there is no particular limitation on the extraction method, and a spitting method, a cotton method, or the like can be used. For example, saliva is extracted by rinsing the mouth with water and then discharging whole saliva to a predetermined container.

Next, the comfort evoking sheet according to the present invention will be described by way of a preferred embodiment thereof. The comfort evoking sheet according to the present embodiment is a sheet that is subjected to the following step (E) in addition to the steps (A) to (D) of the evaluation method of the mode described above and is determined as having comfort evoking performance:

(E) determining, based on a rate of change in the amount of oxytocin or a difference in the amount of oxytocin obtained as a result of the comparison in the step (D), that the test sheet has the ability to evoke comfort by being touched.

That is, the comfort evoking sheet can, by being touched, increase the amount of oxytocin and evoke comfort. With respect to the comfort evoking sheet of the present embodiment, unless inconsistency evokes, the description of the evaluation method of the mode described above is applied as appropriate.

In the step (E), based on the comparison of the amount of oxytocin performed in any one of the steps (D1) to (D3) described above, it is determined that the test sheet has the ability to evoke comfort by being touched. In the comparison of the amount of oxytocin, it may be determined whether or not there is a significant difference. Whether or not there is a significant difference can be checked using the above-described statistical test method such as t-test, Mann-Whitney U test, Wilcoxon signed-rank test, or Dunnett-test.

From the viewpoint of more reliably having comfort evoking performance, the comfort evoking sheet is preferably a comfort evoking sheet that is determined as having comfort evoking performance in the step (E) as a result of comparison between the amount of oxytocin measured in the step (D1) and the amount of oxytocin in the biological sample extracted from the animal before the tactile stimulation is applied, or comparison between the amount of oxytocin measured in the step (D3) and the amount of oxytocin in the biological sample extracted from another population to which the tactile stimulation is not applied. In the evaluation method, in the case where whether or not the sheet has comfort evoking performance is determined in the step (D3) and the step (E), in the step (E), it is preferable to determine the ability to evoke comfort by contact of the test sheet based on the difference in the amount of oxytocin obtained by comparison in the step (D3). In other words, it is preferable to determine that the comfort sheet has comfort evoking performance based on the difference in the amount of oxytocin in the evaluation method that includes the step (D3) and the step (E).

The expression "determining that the sheet has comfort evoking performance" in the step (E) encompasses not only determining whether or not the sheet has comfort evoking performance, but also evaluating the level of comfort evoking performance. That is, the comfort evoking sheet may be a sheet that is determined as having comfort evoking performance and also whose level of comfort evoking performance is evaluated in the step (E).

The inventors of the present invention consider that being fluffy and soft, having a warm feel as well as a smooth and comfortable feel against the skin, and the like contribute to the ability to evoke comfort of the sheet.

Based on the foregoing, from the viewpoint of improving the comfort evoking performance, it is preferable that the comfort evoking sheet has the following physical properties.

The comfort evoking sheet has a compression work WC of preferably 2.0 mN·cm/cm$^2$ or more, more preferably 2.5 mN·cm/cm$^2$ or more, and even more preferably 3.5 mN·cm/cm$^2$ or more, and preferably 20 mN·cm/cm$^2$ or less. The compression work is a measure of cushioning properties of the sheet, and the higher the WC value, the higher the cushioning properties.

The comfort evoking sheet has a compression recovery rate RC of preferably 40% or more, more preferably 46% or more, and even more preferably 52% or more, and preferably 85% or less. The compression recovery rate is a measure that indicates the level of recovery when the sheet is compressed and thereafter decompressed, and the higher the RC value, the higher the compression recovery properties.

The compression work WC and the compression recovery rate RC, as well as average friction coefficient MIU, which will be described later, are measured using KESFB4-AUTO-A (product name) available from Kato Tech Co., Ltd. in accordance with the method described in the following publication.

"The standardization and analysis of hand evaluation" by Sueo Kawabata, 2nd edition, The Textile Machinery Society of Japan, Hand Evaluation and Standardization Committee, published on July 10, 1980

Measurement Method of Compression Work WC and Measurement Method of Compression Recovery Rate RC

A20 cm × 10 cm test piece is cut out from a comfort evoking sheet, and attached to a test stand. Next, the test piece is compressed between steel plates with circular flat surfaces having an area of 2 cm$^2$. The compression rate is set to 0.02 cm/sec, and the maximum compression load is set to 50 g/cm$^2$. The recovery process is also measured at the same rate. The compression work (WC) and the recovery work (WC') are represented by the following equations, respectively. The recovery work (WC') indicates the energy used to recover from the compressed state to the original state. In the equations, $T_m$ indicates the thickness under a load of 49 cN/cm$^2$, and $T_o$ indicates the thickness under a load of 0.49 cN/cm$^2$. Likewise, in the equations, $P_a$ indicates the load (cN/cm$^2$) during measurement (compression process), and $P_b$ indicates the load (cN/cm$^2$) during measurement (recovery process).

The compression recovery rate (RC) is represented by [WC'/WC] × 100, WC'/WC being the ratio between the compression work (WC) during compression and the recovery work (WC') used to recover from the compressed state to the original state.

[Math. 1]

$$WC = \int_{T_0}^{T_m} P_a dT$$

[Math. 2]

$$WC' = -\int_{T_m}^{T_0} P_b dT$$

From the viewpoint of further improving the comfort evoking performance, it is preferable that at least one surface of the comfort evoking sheet has the following surface characteristics.

At least one surface of the comfort evoking sheet has an average friction coefficient MIU of preferably 0.1 or more, and preferably 0.3 or less, and more preferably 0.27 or less.

The average friction coefficient MIU is measured using the following method.

Measurement Method of Average Friction Coefficient MIU

A20 cm × 10 cm test piece is cut out from a comfort evoking sheet, and attached to a test stand with a smooth metal flat surface. Next, the test piece is brought into contact with a contactor, and in that state, the contactor is moved back and forth in the lengthwise direction of the test piece. Specifically, the contact surface of the contactor is pressed against the skin-facing surface of the test piece at a force of 49 cN, and the test piece is moved horizontally by a distance of 2 cm at a constant speed of 0.1 cm/sec. At this time, a uniaxial tension of 19.6 cN/cm is applied to the test piece. The contactor is composed of a U-shaped bundle of 20 piano wires with a diameter of 0.5 mm, the bundle with a width of 10 mm, and the test piece is pressed at a force of 49 cN by a weight. In the back-and-forth movement of the contactor, the friction coefficient when moving back and the friction coefficient when moving forth are measured, the average value is calculated using equation (2) given below, and is defined as average friction coefficient MIU. In the equation (2) given below, the friction coefficient when moving forth is $MIU_{MD1}$, and the friction coefficient when moving back is $MIU_{MD2}$.

$$\text{Average friction coefficient MIU} = \{(MIU_{MD1}{}^2 + MIU_{MD2}{}^2)/2\}^{1/2} \qquad (2)$$

From the viewpoint of improving the feel against the skin, the thickness of the comfort evoking sheet is preferably 1 mm or more, more preferably 1.1 mm or more, and preferably 8 mm or less, more preferably 5 mm or less, and preferably 1 mm or more and 8 mm or less, and more preferably 1.1 mm or more and 5 mm or less.

The thickness of the comfort evoking sheet is measured using the following method.

Measurement Method of Thickness of Comfort evoking Sheet

A sheet that is a measurement target is placed on a horizontal location such that wrinkles and folds are not formed, and the maximum thickness under a load of 5 cN/cm$^2$ is measured as the thickness of the sheet. A thickness gauge PEACOCK DIAL UPRIGHT GAUGES R5-C (available from OZAKI MFG.CO.LTD.) is used to measure the thickness of the sheet. At this time, a plate that is circular or square as viewed in plan view (an acrylic plate with a thickness of about 5 mm) is provided between the tip end portion of the thickness gauge and the measurement portion of the measurement target, and the size of the plate is adjusted such that the load is 5 cN/cm$^2$.

From the viewpoint of obtaining a fluffy and soft feel, it is preferable that one surface of the comfort evoking sheet includes a roughened region having recessed portions and protruding portions. It is preferable that the roughened region is formed such that a plurality of protruding portions protruding from one surface of the sheet and a plurality of recessed portions located between the protruding portions are formed. In this case, the surface from which the protruding portions protrude is preferably skin-facing surface. It is preferable that the sheet including the roughened region is a composite sheet in which a first sheet and a second sheet are stacked and bonded to each other at a plurality of bonding portions, and an area of the first sheet other than the bonding portions protrudes in a direction away from the second sheet, thereby forming the protruding portions. The first and second sheets are preferably fiber sheets, and the fiber sheets are preferably non-woven fabrics, woven fabrics, knitted fabrics, paper, a stack thereof, or the like.

In order to produce the sheet including a roughened region configured as described above, for example, in the same manner as the method disclosed in JP 2015-112343A, a strip-shaped first sheet is supplied between a first roll and a second roll with their circumferential surfaces being engaged with each other to deform the first sheet into a roughened shape, then, the first sheet is moved along the circumferential surface of the first roll from the engaging portion, thereafter, the second sheet is supplied such that the second sheet is overlaid on the first sheet, and the first and second sheets are partially bonded to each other by being sandwiched between the protruding portions of the first roll and a heat roll under heat. At this time, the pattern of the roughened shape of the first roll and the second roll and the pattern of the bonding portions formed by the first roll and the heat roll are different between the center portion and the side portions of the first sheet. It is preferable that, when the first sheet is deformed into a roughened shape by being inserted into the engaging portion between the first roll and the second roll, the first sheet is drawn in a direction of the inside of the roll to facilitate the deformation of the first sheet into a roughened shape.

From the viewpoint of obtaining a fluffy, soft, and warm feel and a comfortable feel against the skin, it is preferable that the comfort evoking sheet is a non-woven fabric, a woven fabric, a knitted fabric, paper, or a stack thereof.

Also, from the viewpoint of obtaining a fluffy, soft, and warm feel and a smooth feel, it is preferable that at least one surface of the comfort evoking sheet is formed using a non-woven fabric.

Examples of the non-woven fabric that can be used for the comfort evoking sheet include an air-through non-woven fabric, a spun-bond non-woven fabric, a spun-lace non-woven fabric, a melt-blown non-woven fabric, a resin bond non-woven fabric, a needle punch non-woven fabric, and the like. It is also possible to use a stack of two or more of the above-listed non-woven fabrics, or a stack of any of the above-listed non-woven fabrics, a film, and the like. Among these, it is preferable to use an air-through non-woven fabric or a spun-bond non-woven fabric.

Also, the non-woven fabric has a basis weight of preferably 10 g/m$^2$ or more, more preferably 15 g/m$^2$ or more, and preferably 40 g/m$^2$ or less, more preferably 35 g/m$^2$ or less, and preferably 10 g/m$^2$ or more and 40 g/m$^2$ or less, and more preferably 15 g/m$^2$ or more and 35 g/m$^2$ or less.

The fibers that constitute the non-woven fabric may be fibers made of thermoplastic resin or the like. Examples of thermoplastic resin include: polyolefins such as polyethylene, polypropylene, and polybden; polyesters such as polyethylene terephthalate, polybutylene terephthalate; polyamides such as nylon 6 and nylon 66; polyacrylic acid, alkyl polymethacrylate, polyvinyl chloride, polyvinylidene chloride, and the like. These resins may be used alone or as a blend composed of a combination of two or more. Also, the composite fiber may be used in the form of sheath-core fiber, or side-by-side fiber.

From the viewpoint of improving the comfort evoking performance, the fibers that constitute one surface of the non-woven fabric have a fiber fineness of preferably 0.5 dtex or more, preferably 3.3 dtex or less, and more preferably 2.5 dtex or less. As a result of one surface of the non-woven fabric being made of the above-described fibers, or in other words, as a result of the fibers being exposed from at least a portion of the surface of the non-woven fabric, a more pleasant feel can be provided. The fiber fineness of the fibers is measured as described below.

Measurement Method of Fiber Fineness

A measurement sample is produced by cutting out a 50 mm × 100 mm rectangular piece (with an area of 5000 mm$^2$) from a non-woven fabric under no load. Next, a cross section of the measurement sample is viewed to measure the fiber thickness of 10 fibers at a position 0.2 mm spaced apart from one surface in the thickness direction using an electron microscope, and average fiber thickness value Dn ($\mu$m) is calculated. Next, the resin constituting the fibers at a position 0.2 mm spaced apart from the one surface in the thickness direction is identified, and theoretical fiber density Pn (g/cm$^3$) is determined using a differential scanning calorimeter (DSC). From the average fiber thickness value Dn ($\mu$m) and theoretical fiber density Pn (g/cm$^3$) that were obtained, weight (g) per a fiber length of 10,000 m is calculated, and the calculated values is defined as the fiber fineness (dtex) of the fibers constituting the one surface.

The comfort evoking sheet described above can increase the amount of oxytocin and evoke comfort by being touched. With an article in which such a comfort evoking sheet is used, when either one surface of the sheet is provided to be capable of being brought into contact with the human skin, the article can evoke comfort to a user when the user touches the article.

The article in which the comfort evoking sheet is used is preferably an article that can be brought into contact with the skin or hairs of the user. Examples of the article include clothes, accessories, appliances, bedclothes, covers, toys, and the like.

Examples of the clothes include underwear, underclothes, shirts, overcoats, hoodies, skirts, blousons, dresses, jackets, pants, sweatshirts, absorbent articles such as a disposable diaper, and the like. The area of the clothes that is provided to be capable of being brought into contact with the human skin is, for example, the outer surface, the back surface, or the like of the clothes. The absorbent article is used to absorb and retain body fluids discharged from the body such as urine and menstrual blood. The absorbent article encompasses a disposable diaper, a sanitary napkin, an incontinence pad, a panty liner, and the like, but the absorbent article is not limited thereto. The absorbent article broadly encompasses articles that are used to absorb fluids discharged from the human body. Typically, the absorbent article includes, as with a diaper 10 shown in Fig. 2, a top sheet, a back sheet, and a liquid retainable absorbent member that is provided between the top sheet and the back sheet. Other than the above, the absorbent article may include an outer member that is provided on the non-skin-facing surface side relative to the back sheet and forms an outer surface of the absorbent article. The absorbent article may further include various types of members according to its specific application. Such members are known to those having ordinary skill in the art. The area of the absorbent article that is provided to be capable of being brought into contact with the human skin is formed using a constituent member that is brought into contact with the skin. The constituent member that is brought into contact with the skin is a constituent member that is brought into contact with the skin of a user, a parent or the like of the user, or the like. The constituent member may be, for example, a top sheet, an outer member, or the like.

Examples of the accessories include: accessories such as a bracelet; footwear such as shoes, sandals, and slippers; and equipment such as socks, gloves, belts, headwear, belly bands, scarfs, and stoles. Other examples include shoe insoles, gloves, and the backing material of headwear or the like. The area of the accessories that is provided to be capable of being brought into contact with the human skin is, for example, the back surface of a shoe insole, a sock, a glove, or headwear, or the like.

Examples of the appliances include: medical appliances that are used to support a joint such as the elbow or the wrist, to relieve pain, as well as for rehabilitation, medical treatment, and the like; assisting appliances that are used to correct posture as well as for sports, and the like; and the like. Examples of the medical appliances include a joint protector, a patch, and hygiene products such as a bandage, an eye bandage, a gauze bandage, and a mask. Examples of the assisting appliances include a corset, a supporter, and the like. The area of appliances that is provided to be capable of being brought into contact with the human skin is, for example, the back surface of a shoe insole, a sock, a glove, or headwear, or the like.

Examples of the bedclothes include a futon, a mat, a mattress, a bed, a cushion, a pillow, and the like. Examples of the futon include a blanket, a comforter, a coverlet for a *kotatsu* (Japanese heater equipped table), and the like.

Examples of the covers include a futon cover, a cushion cover, a pillowcase, a bed sheet, a sofa cover, a *zabuton* (Japanese floor cushion) cover, an automobile seat cover, a lavatory seat cover, and the like. The term "bed sheet" means anything that covers a mat, a mattress, a bed, or the like.

Examples of the toys include stuffed toys, clothes for stuffed toys, accessories, and the like.

The area of the bedclothes, the covers, or the toys that is provided to be capable of being brought into contact with the human skin is, for example, the surface of the bedclothes, the covers, or the toys.

Also, the article in which the comfort evoking sheet is used may be a product other than the articles described above. Examples of the product other than the article described above include a handkerchief, a towel, a place mat, a tablecloth, and the like.

Increasing the amount of oxytocin in babies and infants is considered to be effective in forming affection between the parents and the babies and infants as well as development of the babies and infants. From this viewpoint, the comfort evoking sheet is preferably used in an article for babies and infants. In this case, it is preferable to provide the comfort evoking sheet in an article for babies and infants such that either one surface of the comfort evoking sheet is provided to be capable of being brought into contact with the skin, from the viewpoint of giving comfort to a baby or an infant who has touched the article and a parent or the like of the baby of the infant.

The article for babies and infants in which the comfort evoking sheet is used is preferably an article for babies and infants that can be brought into contact with the skin or hairs. Examples of the article include clothes, accessories, bedclothes, covers, toys, and the like for babies and infants. The clothes, accessories, bedclothes, covers and toys are those designed for babies and infants, and those given as examples can be used unless inconsistency evokes.

Also, the clothes for babies and infants are those designed for babies and infants, and a coverall can be used in addition to those given as examples.

The accessories for babies and infants are those designed for babies and infants, and equipment such as a baby bib, a baby wrapper, and a pair of mittens can be used in addition to those given as examples.

Examples of the covers for babies and infants include a stroller seat cover, a child car seat cover, and the like.

It is preferable to attach an information label to the article and the article for babies and infants described above, and the packaging thereof, the information label being provided to inform people who buy or use the article of the fact that the article includes a sheet with comfort evoking performance or that the article can evoke comfort. As the information label, an information label that contains text such as "dad, mom, and baby are happy" and "with the function of increasing the amount of oxytocin", and an illustration that indicates the content of the text may be provided by a method such as printing.

Up to here, the present invention has been described by way of a preferred mode and embodiment thereof, but the present invention is not limited to the embodiment given above, and may be changed as appropriate.

Also, the evaluation method according to the present invention described above is preferably used in a method for designing an absorbent article, wherein a sheet selected based on an evaluation result of the evaluation method is determined as a constituent member that is brought into contact with the skin. As described above, the constituent member that is brought into contact with the skin is a constituent member such as, for example, a top sheet or an outer member. The sheet selected based on an evaluation result is, for example, the comfort evoking sheet described above. In the method for designing an absorbent article, it is preferable to perform evaluation on a plurality of sheets using the evaluation method according to the present invention, and select a comfort evoking sheet from among the plurality of sheets based on a result of the evaluation.

Also, the sheet selected as a result of performing the designing method is used in a method for producing an absorbent article. That is, the absorbent article designed using the designing method described above is produced using the selected sheet. The absorbent article obtained using the production method can evoke comfort to a user such as a wearer who has touched the absorbent article, or a parent of the wearer.

With respect to the mode and the embodiment described above, the present invention further discloses a method for evaluating comfort evoking performance of sheets, a comfort evoking sheet, and an article described below.

<1>

A method for evaluating comfort evoking performance of sheets, with which whether or not a sheet has an ability to evoke comfort as a result of the sheet being touched, or a level of the ability is evaluated, the method including the following steps (A) to (D):

(A) bringing a test sheet, which is an evaluation target, into contact with the skin or hairs of an animal and applying a tactile stimulation to the animal;
(B) extracting a biological sample from the animal after the tactile stimulation has been applied;
(C) measuring an amount of oxytocin in the biological sample; and

(D) comparing the amount of oxytocin measured with an amount of oxytocin in a biological sample extracted from the animal under a condition where there is no effect of the tactile stimulation.

<2>

The evaluation method as set forth in clause <1>,
wherein, in the step (B), the biological sample is extracted from the animal within 60 minutes after the tactile stimulation has been applied.

<3>

The evaluation method as set forth in clause <1> or <2>,
wherein the test sheet does not contain oxytocin or an oxytocin inducing agent.

<4>

The evaluation method as set forth in any one of clauses <1> to <3>,
wherein a time when there is no effect of the tactile stimulation is before the tactile stimulation is applied.

<5>

The evaluation method as set forth in clause <4>,
wherein a timing at which the biological sample is extracted before the tactile stimulation is applied by the test sheet being touched is during a period from 30 minutes to immediately before the tactile stimulation is applied, and preferably during a period from 15 minutes to immediately before the tactile stimulation is applied, and
it takes 5 minutes or more and 15 minutes or less, and preferably 10 minutes to extract the biological sample.

<6>

The evaluation method as set forth in any one of clauses <1> to <5>,
wherein, in the step (D), if a rate of change in the amount of oxytocin measured in the step (C) relative to the amount of oxytocin in the biological sample extracted from the animal under the condition where there is no effect of the tactile stimulation increases by 10% or more, and preferably by 30% or more, it is determined that the test sheet has the comfort evoking performance.

<7>

The evaluation method as set forth in clause <6>,
wherein, in the step (D), the following step (D1) or (D2) is carried out:

(D1) comparing the amount of oxytocin measured with an amount of oxytocin in a biological sample extracted from the animal before the tactile stimulation is applied; or
(D2) comparing the amount of oxytocin measured with an amount of oxytocin in a biological sample extracted from the animal at a time when the amount of oxytocin no longer varies due to the tactile stimulation after the tactile stimulation is applied, and

if the amount of oxytocin measured in the step (C) increases by 10% or more, and preferably by 30% more, relative to the amount of oxytocin in the biological sample extracted from the animal under the condition where there is no effect of the tactile stimulation, it is determined that the test sheet has the comfort evoking performance.

<8>

The evaluation method as set forth in clause <6> or <7>,
wherein, in the step (D), the following step (D3) is carried out:

(D3) comparing the amount of oxytocin measured that is an amount of oxytocin in a biological sample extracted from a population to which the tactile stimulation is applied with an amount of oxytocin in a biological sample extracted from another population to which the tactile stimulation is not applied, and

in the step (D3), if the amount of oxytocin in the biological sample extracted from the population to which the tactile stimulation is applied increases by 10% or more, and preferably by 30% or more, relative to the amount of oxytocin in the biological sample extracted from the population to which the tactile stimulation is not applied, it is determined that the test sheet has the comfort evoking performance.

<9>

The evaluation method as set forth in any one of clauses <1> to <8>,
wherein, in the step (A), the tactile stimulation is applied to the animal by moving a haired portion or a non-haired portion on the skin of the animal while it is in contact with a surface of the test sheet.

<10>

The evaluation method as set forth in clause <9>,
wherein the non-haired portion is a palm.

<11>

The evaluation method as set forth in any one of clauses <1> to <10>,
wherein, in the step (A), the tactile stimulation is applied to the animal continuously or intermittently for 30 seconds or more.

<12>

The evaluation method as set forth in clause <11>,
wherein, in the step (A), a length of time during which the tactile stimulation is applied to the animal continuously or intermittently is 30 seconds or more, preferably 45 seconds or more, and more preferably 60 seconds or more, and 600 seconds or less, preferably 450 seconds or less, and more preferably 300 seconds or less, and also 30 seconds or more and 600 seconds or less, preferably 45 seconds or more and 450 seconds or less, and more preferably 60 seconds or more and 300 seconds or less.

<13>

The evaluation method as set forth in clause <11>,
wherein, in the step (A), the tactile stimulation is applied to the animal continuously for 30 seconds or more.

<14>

The evaluation method as set forth in clause <11>,
wherein, in the step (A), a length of time during which the tactile stimulation is applied to the animal continuously is 30 seconds or more, preferably 45 seconds or more, and more preferably 60 seconds or more, and 300 seconds or less, preferably 240 seconds or less, more preferably 180 seconds or less, and also 30 seconds or more and 300 seconds or less, preferably 45 seconds or more and 240 seconds or less, and more preferably 60 seconds or more and 180 seconds or less.

<15>

The evaluation method as set forth in clause <11>,
wherein, in the step (A), the tactile stimulation is applied to the animal intermittently by repeating a cycle of applying the tactile stimulation and maintaining a resting state,
a length of time during which the tactile stimulation is applied is 15 seconds or more and 180 seconds or less, and preferably 30 seconds or more and 120 seconds or less,
a length of time during which the resting state is maintained is 15 seconds or more and 180 seconds or less, and preferably 30 seconds or more and 120 seconds or less.

<16>

The evaluation method as set forth in any one of clauses <1> to <15>,
wherein the biological sample is blood, blood serum, blood plasma, urine, or saliva.

<17>

A method for designing an absorbent article, the method including
performing evaluation on a plurality of sheets using the evaluation method as set forth in any one of clauses <1> to <16>, and determining a sheet selected based on a result of the evaluation as a constituent member that is brought into contact with skin.

<18>

A method for producing an absorbent article, the method including
producing an absorbent article designed based on the designing method as set forth in clause <17> by using the selected sheet.

<19>

A comfort evoking sheet that is determined as having an ability to evoke comfort by being touched, using a method for evaluating a sheet including the following steps (A) to (C), (D), and (E),

(A) bringing a test sheet, which is an evaluation target, into contact with the skin or hairs of an animal and applying a tactile stimulation to the animal;
(B) extracting a biological sample from the animal after the tactile stimulation has been applied;
(C) measuring an amount of oxytocin in the biological sample;
(D) comparing the amount of oxytocin measured with an amount of oxytocin in a biological sample extracted from the animal under a condition where there is no effect of the tactile stimulation; and
(E) determining, based on a rate of change in the amount of oxytocin or a difference in the amount of oxytocin obtained as a result of the comparison in the step (D), that the test sheet has the ability to evoke comfort by being touched.

<20 >

The comfort evoking sheet as set forth in clause <19>,
wherein, in the step (B), the biological sample is extracted from the animal within 60 minutes after the tactile stimulation has been applied.

<21>

The comfort evoking sheet as set forth in clause <19> or <20>,
wherein, in the step (D), the amount of oxytocin measured that is an amount of oxytocin in a biological sample extracted from a population to which the tactile stimulation is applied is compared with an amount of oxytocin in a biological sample extracted from another population to which the tactile stimulation is not applied, and
in the step (E), it is determined, based on a difference in the amount of oxytocin obtained as a result of the comparison in the step (D), that the test sheet has the ability to evoke comfort by being touched.

<22>

The comfort evoking sheet as set forth in any one of clauses <19> to <21>,
wherein the comfort evoking sheet has a compression work of 2.0 mN·cm/cm$^2$ or more, preferably 2.5 mN·cm/cm$^2$ or more, and more preferably 3.5 mN·cm/cm$^2$ or more, and 20 mN·cm/cm$^2$ or less.

<23>

The comfort evoking sheet as set forth in any one of clauses <19> to <22>,
wherein the comfort evoking sheet has a compression recovery rate RC of 40% or more, preferably 46% or more, more preferably 52% or more, and 85% or less.

<24>

The comfort evoking sheet as set forth in clause <22> or <23>,

wherein the comfort evoking sheet has a compression work of 2.0 mN·cm/cm$^2$ or more and a compression recovery rate of 40% or more.

<25>

The comfort evoking sheet as set forth in any one of clauses <19> to <24>,
wherein at least one surface of the comfort evoking sheet has an average friction coefficient MIU of 0.3 or less.

<26>

The comfort evoking sheet as set forth in clause <25>,
wherein the at least one surface of the comfort evoking sheet has an average friction coefficient MIU of 0.1 or more and 0.3 or less, and preferably 0.27 or less.

<27>

The comfort evoking sheet as set forth in any one of clauses <19> to <26>,
wherein the comfort evoking sheet has a thickness of 1 mm or more.

<28>

The comfort evoking sheet as set forth in clause <27>,
wherein the comfort evoking sheet has a thickness of 1 mm or more, preferably 1.1 mm or more, and 8 mm or less, preferably 5 mm or less, and also 1 mm or more and 8 mm or less, and preferably 1.1 mm or more and 5 mm or less.

<29>

The comfort evoking sheet as set forth in any one of clauses <19> to <28>,
wherein the comfort evoking sheet includes, on one surface, a roughened region that includes recessed portions and protruding portions.

<30>

The comfort evoking sheet as set forth in any one of clauses <19> to <29>,
wherein the comfort evoking sheet is a non-woven fabric, a woven fabric, a knitted fabric, paper, or a stack thereof.

<31>

The comfort evoking sheet as set forth in any one of clauses <19> to <30>,
wherein at least one surface of the comfort evoking sheet is formed using a non-woven fabric.

<32>

The comfort evoking sheet as set forth in clause <30> or <31>,
wherein the comfort evoking sheet contains fibers with a fiber fineness of 3.3 dtex or less, and a portion of the fibers is exposed to a portion of a surface of the sheet.

<33>

An article in which the comfort evoking sheet as set forth in any one of clauses <19> to <32> is used,
wherein either one surface of the sheet is provided so as to be capable of coming into contact with the skin of a human, and
the article is any one of clothes, accessories, appliances, bedclothes, covers, and toys.

<34>

An article for babies and infants in which the comfort evoking sheet as set forth in any one of clauses <19> to <32> is used,
wherein either one surface of the sheet is provided to be capable of being brought into contact with the skin of a human, and

the article is any one of clothes, accessories, diapers, bedclothes, and toys.

Examples

Hereinafter, the present invention will be described in further detail by way of examples, but the present invention is not limited to the examples given below.

Example 1

A roughened sheet was produced with a roughened region in which recessed portions are protruding portions were formed. The roughened sheet was produced based on the method described above by overlaying a second sheet on a first sheet deformed into a roughened shape, and bonding the first sheet and the second sheet through thermal fusion. At this time, a roughened sheet with large protruding portions and recessed portions was produced by adjusting the engaging depth between the first roll and the second roll. The recessed portions were formed in an area other than the protruding portions. As the first and second sheets, non-woven fabrics produced by an air-through method using a sheath-core fiber whose core component was polyethylene terephthalate (PET) and whose sheath component was polyethylene (PE), the non-woven fabrics having a basis weight of 18 g/m$^2$, were used. As the second sheet, a non-woven fabric produced by an air-through method using a sheath-core fiber whose core component was polyethylene terephthalate (PET) and whose sheath component was polyethylene (PE), the non-woven fabrics having a basis weight of 18 g/m$^2$, was used.

Example 2

A roughened sheet with medium protruding portions having a height lower than those of Example 1 and recessed portions was produced by adjusting the engaging depth between the first roll and the second roll. A sheet was produced in the same manner as Example 1, except for the engaging depth between the first roll and the second roll.

Example 3

A roughened sheet with small protruding portions having a height lower than those of Example 2 and recessed portions was produced by adjusting the engaging depth between the first roll and the second roll. A sheet was produced in the same manner as Example 1, except for the engaging depth between the first roll and the second roll.

Comparative Example 1

A flat sheet with no recessed portions and protruding portions was produced. A sheet was produced in the same manner as in Example 1, except that the first sheet was not deformed into a roughened shape.
For each of the sheets produced in Examples 1 to 3 and Comparative Example 1, the range of movement of the tip of protruding portions, the surface characteristics of the skin-facing surface of the sheet, and the physical properties of the sheet are shown in Table 1 given below. These measurements are obtained using the methods described above. As used herein, the skin-facing surface of the sheet refers to a surface that comes into contact with a hand of a panelist during measurement of the amount of oxytocin, which will be described later.

Measurement of Amount of Oxytocin

Each of the sheets produced in Examples 1 to 3 and Comparative Example 1 was used as the top sheet of a diaper. Each sheet was incorporated in a diaper such that the lengthwise direction of the sheet matched the lengthwise direction of the diaper. The diaper was placed such that the surface of the top sheet with protruding portions faced upward. Next, the sheet was touched by 10 healthy women panelists in their twenties to thirties to apply a tactile stimulation to the sheet, and saliva of each panelist was extracted before and after the tactile stimulation had been applied as a biological sample. As shown in Fig. 2, each panelist touched the top sheet of the diaper with the palms of both hands so as to apply a tactile stimulation. Also, a cycle of touching the top sheet with the palms for 30 seconds to apply a tactile stimulation and maintaining a resting state in which the top sheet was left without being touched for 30 seconds was repeated 5 times. Immediately after the application of the tactile stimulation, the mouth is rinsed with water, then, whole saliva in the mouth was discharged to a centrifuge tube (with a volume of 50 mL) over 10 minutes, and the discharged saliva was defined as "the biological sample obtained 0 to 10 minutes after the tactile stimulation has been applied". In the same manner, 30 minutes before the tactile stimulation was applied, saliva was extracted over 10 minutes, and the extracted saliva was defined as "the biological sample obtained before the tactile stimulation was applied". Likewise, after "the biological sample obtained 0 to 10 minutes after the tactile stimulation has been applied" had been extracted, the biological sample was left still for 20 minutes. Then, saliva was extracted in the same manner, and the extracted saliva was defined as "the biological sample obtained 30 to 40 minutes after the tactile stimulation has been applied". The extracted saliva was rapidly cooled using dry ice, and thereafter stored at -80°C.

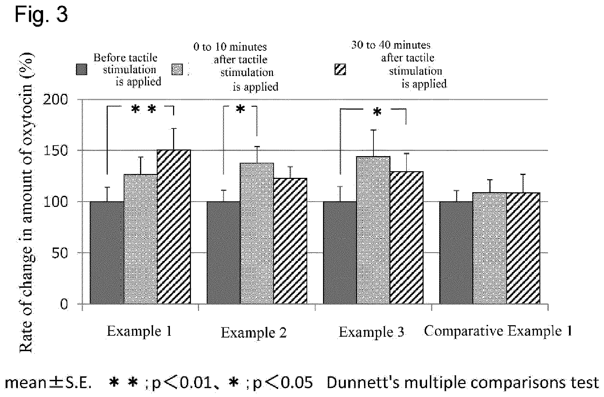

The extracted saliva was centrifuged at 15,000 rpm for 10 minutes, and thereafter a supernatant was extracted, and was mixed with 0.1% (v/v) trifluoroacetic acid (TFA) in an amount equal to that of the supernatant. The resulting mixture was centrifuged at 3,000 rpm for 30 minutes so as to extract a supernatant. The supernatant was subjected to a Sep-pak C18 column (200 mg, 3 cc, Waters), and extraction was performed in the following manner. 1 mL of 100% acetonitrile (ACN) was allowed to pass through the C18 column, and then 10 mL of 0.1% TFA solution (v/v) was allowed to pass through the C18 column. After that, whole saliva (3.0 to 6.0 mL) mixed with a 0.1% TFA solution (v/v) was allowed to pass through the C18 column, and washing was performed using 10 mL of 0.1% TFA solution (v/v), and thereafter the solution was eluted using 3 mL (95% CAN / 5% (0.1% TFA solution)) (v/v). The CAN contained in the eluted solution was evaporated using $N_2$ gas, and the remaining aqueous solution was freeze dried. The obtained freeze dried product was dissolved in 250 $\mu$L of Assay Buffer included in an Oxytocin ELISA kit (Enzo), and the amount of oxytocin in the saliva was quantified using the kit. Then, the average value of the amount of oxytocin in the biological sample before the tactile stimulation was applied was set to 100%, and the rate of change in the amount of oxytocin in the biological sample after the tactile stimulation had been applied with respect the average value was obtained. Also, whether or not there was a significant difference in the amount of oxytocin before and after the tactile stimulation was applied was checked using a Dunnett-test. Fig. 3 shows a graph of the amount of oxytocin before and after the tactile stimulation was applied.

Evaluation of Feel Against the Skin

In Measurement of Amount of Oxytocin section, after saliva was extracted immediately after the tactile stimulation had been applied, the sheet was evaluated in terms of the feel against the skin by the panelists based on the following evaluation scale: 1 to 7. The average values of 10 panelists were used as evaluation results, with "Neither good nor bad" being set in the middle of the scale, and 3 rating levels on "Very good" side and 3 rating levels on "Very bad" side. The evaluation results are shown in Table 1 given below.

Very good: 7 points

Good: 6 points

Slightly good: 5 points

Neither good nor bad: 4 points

Slightly bad: 3 points

Bad: 2 points

Very bad: 1 point

[Table 1]

| | | Ex. 1 | Ex. 2 | Ex. 3 | Comp. Ex. 1 |
|---|---|---|---|---|---|
| Compression characteristics | Compression work WC (mN·cm/cm$^2$) | 4.20 | 4.40 | 2.20 | 0.5 |
| | Compression recovery rate RC (%) | 73.2 | 68.1 | 46.2 | 48.4 |
| Surface characteristics of skin-facing surface | Average friction coefficient MIU | 0.22 | 0.26 | 0.25 | 0.16 |
| Evaluation of the feel against skin | | 7 | 6 | 5 | 3 |

From the results shown in Fig. 3, it can be seen that, with the sheets of Examples 1 to 3, the amount of oxytocin increased significantly upon application of the tactile stimulation. That is, the effect of evoking comfort upon application of the tactile stimulation can be expected. Also, with the sheets of Examples 1 to 3, "Very good", "Good" or "Slightly good" were obtained in terms of the feel against the skin. On the other hand, with the sheet of Comparative Example 1, no change was observed in the amount of oxytocin before and after the tactile stimulation was applied, and the evaluation result of the feel against the skin was not good.

Industrial Applicability

According to the present invention, comfort evoking performance that can increase the amount of oxytocin as a result of the tactile stimulation being applied to the sheet can be evaluated objectively and quantitatively. Also, the present invention can provide a method for designing an absorbent article and a method for producing the absorbent article based on the evaluation result thereof. Also, the present invention can provide a sheet with the ability to increase the amount of oxytocin without using oxytocin itself or an oxytocin inducing substance, and an article or an article for babies and infants in which the sheet is used.

**Claims**

1. A method for evaluating comfort evoking performance of sheets, with which whether or not a sheet has an ability to evoke comfort as a result of the sheet being touched, or a level of the ability is evaluated, the method comprising the following steps (A) to (D):

   (A) bringing a test sheet, which is an evaluation target, into contact with the skin or hairs of an animal and applying a tactile stimulation to the animal;
   (B) extracting a biological sample from the animal after the tactile stimulation has been applied;
   (C) measuring an amount of oxytocin in the biological sample; and
   (D) comparing the amount of oxytocin measured with an amount of oxytocin in a biological sample extracted from the animal under a condition where there is no effect of the tactile stimulation.

2. The evaluation method according to claim 1,
   wherein, in the step (B), the biological sample is extracted from the animal within 60 minutes after the tactile stimulation has been applied.

3. The evaluation method according to claim 1 or 2,
   wherein the test sheet does not contain oxytocin or an oxytocin inducing agent.

4. The evaluation method according to any one of claims 1 to 3,
   wherein a time when there is no effect of the tactile stimulation is before the tactile stimulation is applied.

5. The evaluation method according to claim 4,
   wherein a timing at which the biological sample is extracted before the tactile stimulation is applied by the test sheet being touched is during a period from 30 minutes to immediately before the tactile stimulation is applied, and
   it takes 5 minutes or more and 15 minutes or less to extract the biological sample.

6. The evaluation method according to any one of claims 1 to 5,
   wherein, in the step (D), if a rate of change in the amount of oxytocin measured in the step (C) relative to the amount of oxytocin in the biological sample extracted from the animal under the condition where there is no effect of the tactile stimulation increases by 10% or more, it is determined that the test sheet has the comfort evoking performance.

7. The evaluation method according to claim 6,
   wherein, in the step (D), the following step (D1) or (D2) is carried out:

   (D1) comparing the amount of oxytocin measured with an amount of oxytocin in a biological sample extracted from the animal before the tactile stimulation is applied; or
   (D2) comparing the amount of oxytocin measured with an amount of oxytocin in a biological sample extracted from the animal at a time when the amount of oxytocin no longer varies due to the tactile stimulation after the tactile stimulation is applied, and
   if the amount of oxytocin measured in the step (C) increases by 10% or more relative to the amount of oxytocin in the biological sample extracted from the animal under the condition where there is no effect of the tactile stimulation, it is determined that the test sheet has the comfort evoking performance.

8. The evaluation method according to claim 6 or 7,
wherein, in the step (D), the following step (D3) is carried out:

(D3) comparing the amount of oxytocin measured that is an amount of oxytocin in a biological sample extracted from a population to which the tactile stimulation is applied with an amount of oxytocin in a biological sample extracted from another population to which the tactile stimulation is not applied, and
in the step (D3), if the amount of oxytocin in the biological sample extracted from the population to which the tactile stimulation is applied increases by 10% or more relative to the amount of oxytocin in the biological sample extracted from the population to which the tactile stimulation is not applied, it is determined that the test sheet has the comfort evoking performance.

9. The evaluation method according to any one of claims 1 to 8,
wherein, in the step (A), the tactile stimulation is applied to the animal by moving a haired portion or a non-haired portion on the skin of the animal while it is in contact with a surface of the test sheet.

10. The evaluation method according to claim 9,
wherein the non-haired portion is a palm.

11. The evaluation method according to any one of claims 1 to 10,
wherein, in the step (A), the tactile stimulation is applied to the animal continuously or intermittently for 30 seconds or more.

12. The evaluation method according to claim 11,
wherein, in the step (A), a length of time during which the tactile stimulation is applied to the animal continuously or intermittently is 30 seconds or more and 600 seconds or less.

13. The evaluation method according to claim 11,
wherein, in the step (A), the tactile stimulation is applied to the animal continuously for 30 seconds or more.

14. The evaluation method according to claim 11,
wherein, in the step (A), a length of time during which the tactile stimulation is applied to the animal continuously is 30 seconds or more and 300 seconds or less.

15. The evaluation method according to claim 11,
wherein, in the step (A), the tactile stimulation is applied to the animal intermittently by repeating a cycle of applying the tactile stimulation and maintaining a resting state,
a length of time during which the tactile stimulation is applied is 15 seconds or more and 180 seconds or less, and
a length of time during which the resting state is maintained is 15 seconds or more and 180 seconds or less.

16. The evaluation method according to any one of claims 1 to 15,
wherein the biological sample is blood, blood serum, blood plasma, urine, or saliva.

17. A method for designing an absorbent article, the method comprising
performing evaluation on a plurality of sheets using the evaluation method according to any one of claims 1 to 16, and determining a sheet selected based on a result of the evaluation as a constituent member that is brought into contact with skin.

18. A method for producing an absorbent article, the method comprising
producing an absorbent article designed based on the designing method according to claim 17 by using the selected sheet.

19. A comfort evoking sheet that is determined as having an ability to evoke comfort by being touched, using a method for evaluating a sheet comprising the following steps (A) to (C), (D), and (E),

(A) bringing a test sheet, which is an evaluation target, into contact with the skin or hairs of an animal and applying a tactile stimulation to the animal;
(B) extracting a biological sample from the animal after the tactile stimulation has been applied;
(C) measuring an amount of oxytocin in the biological sample;
(D) comparing the amount of oxytocin measured with an amount of oxytocin in a biological sample extracted from the animal under a condition where there is no effect of the tactile stimulation; and
(E) determining, based on a rate of change in the amount of oxytocin or a difference in the amount of oxytocin obtained as a result of the comparison in the step (D), that the test sheet has the ability to evoke comfort by being touched.

20. The comfort evoking sheet according to claim 19,
wherein, in the step (B), the biological sample is extracted from the animal within 60 minutes after the tactile stimulation has been applied.

21. The comfort evoking sheet according to claim 19 or 20,
wherein, in the step (D), the amount of oxytocin measured that is an amount of oxytocin in a biological sample extracted from a population to which the tactile stimulation is applied is compared with an amount of oxytocin in a biological sample extracted from another population to which the tactile stimulation is not applied, and
in the step (E), it is determined, based on a difference in the amount of oxytocin obtained as a result of the comparison in the step (D), that the test sheet has the ability to evoke comfort by being touched.

22. The comfort evoking sheet according to any one of claims 19 to 21,
wherein the comfort evoking sheet has a compression work of 2.0 mN·cm/cm$^2$ or more and 20 mN·cm/cm$^2$ or less.

23. The comfort evoking sheet according to any one of claims 19 to 22,
wherein the comfort evoking sheet has a compression recovery rate RC of 40% or more and 85% or less.

24. The comfort evoking sheet according to claim 22 or 23,
wherein the comfort evoking sheet has a compression work of 2.0 mN·cm/cm$^2$ or more and a compression recovery rate of 40% or more.

25. The comfort evoking sheet according to any one of claims 19 to 24,
wherein at least one surface of the comfort evoking sheet has an average friction coefficient MIU of 0.3 or less.

26. The comfort evoking sheet according to claim 25,
wherein the at least one surface of the comfort evoking sheet has an average friction coefficient MIU of 0.1 or more and 0.3 or less.

27. The comfort evoking sheet according to any one of claims 19 to 26,
wherein the comfort evoking sheet has a thickness of 1 mm or more.

28. The comfort evoking sheet according to claim 27,
wherein the comfort evoking sheet has a thickness of 1 mm or more and 8 mm or less.

29. The comfort evoking sheet according to any one of claims 19 to 28,
wherein the comfort evoking sheet includes, on one surface, a roughened region that includes recessed portions and protruding portions.

30. The comfort evoking sheet according to any one of claims 19 to 29,
wherein the comfort evoking sheet is a non-woven fabric, a woven fabric, a knitted fabric, paper, or a stack thereof.

31. The comfort evoking sheet according to any one of claims 19 to 30,
wherein at least one surface of the comfort evoking sheet is formed using a non-woven fabric.

32. The comfort evoking sheet according to claim 30 or 31,
wherein the comfort evoking sheet contains fibers with a fiber fineness of 3.3 dtex or less, and a portion of the fibers is exposed to a portion of a surface of the sheet.

33. An article in which the comfort evoking sheet according to any one of claims 19 to 32 is used,

wherein either one surface of the sheet is provided so as to be capable of coming into contact with the skin of a human, and
the article is any one of clothes, accessories, appliances, bedclothes, covers, and toys.

**34.** An article for babies and infants in which the comfort evoking sheet according to any one of claims 19 to 32 is used, wherein either one surface of the sheet is provided to be capable of being brought into contact with the skin of a human, and
the article is any one of clothes, accessories, diapers, bedclothes, and toys.

Fig. 1

Fig. 2

# Fig. 3

mean±S.E.  **;p<0.01、*;p<0.05  Dunnett's multiple comparisons test

<div align="center">

**INTERNATIONAL SEARCH REPORT**

</div>

| | International application No. |
|---|---|
| | PCT/JP2019/020888 |

**A. CLASSIFICATION OF SUBJECT MATTER**
Int.Cl. G01N33/74(2006.01)i, A61F13/511(2006.01)i,
A61F13/84(2006.01)i, G01N33/36(2006.01)i

According to International Patent Classification (IPC) or to both national classification and IPC

**B. FIELDS SEARCHED**

Minimum documentation searched (classification system followed by classification symbols)
Int.Cl. G01N33/74, A61F13/511, A61F13/84, G01N33/36

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2014-079291 A (KAO CORP.) 08 May 2014, claim 1, paragraphs [0009], [0024]-[0026], [0041] (Family: none) | 19-24,27-34<br>1-18,25-26 |
| X<br><br>A | JP 2005-000428 A (DAIO PAPER CORP.) 06 January 2005, paragraphs [0029]-[0030], tables 11, 12, paragraph [0062] (Family: none) | 19-24,27-31,<br>33-34<br>1-18,25-26,32 |

☒ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 August 2019 (15.08.2019) | 27 August 209 (27.08.2019) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

**INTERNATIONAL SEARCH REPORT**

| International application No. |
|---|
| PCT/JP2019/020888 |

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2016-220752 A (NIPPON PAPER CRECIA CO., LTD.) 28 December 2016, claims 1-2, paragraphs [0008], [0015]-[0018], [0028]-[0033] (Family: none) | 19-21,25-34<br>1-18,22-24 |
| X<br><br>A | JP 2005-348938 A (DAIO PAPER CORP.) 22 December 2005, claims 1, 5 & US 2008/0294135 A1, claims 1, 5 & EP 1757255 A1 & CN 1964685 A | 19-21,25-31,33-34<br>1-18,23-24,32 |
| A | JP 2010-117232 A (SHISEIDO CO., LTD.) 27 May 2010, claims 1-2, paragraphs [0018]-[0030] (Family: none) | 1-34 |
| A | WO 2014/092087 A1 (UNI-CHARM CORP.) 19 June 2014, claims 1-11 & CN 104837449 A, claims 1-11 & TW 201440742 A & AU 2013358099 A | 1-34 |
| A | RIEM, M. M. E. et al., "Emotional maltreatment is associated with atypical responding to stimulation of endogenous oxytocin release through mechanically-delivered massage in males", Psychoneuroendocrinology, vol. 85, 2017, pp. 115-122 | 1-34 |
| A | WALKER, S. C. et al., "C-tactile afferents: Cutaneous mediators of oxytocin release during affiliative tactile interactions?", Neuropeptides, 2017, vol. 64, pp. 27-38 | 1-34 |
| E,A | JP 2019-132822 A (KAO CORP.) 08 August 2019, claims 1-5 (Family: none) | 1-34 |
| P,X | 坂本考司 他, 手の平（無毛部）で物に触れることによる親子間の愛着形成に関わる生理的変化, 母性衛生, September 2018, vol. 59, no. 3, p. 312, P2-089, non-official translation (SAKAMOTO, Takashi et al., "Physiological changes related to parent-child relation attachment formation  by touching objects with the palm (hairless part)", Maternal health) | 1-5,9-18,19-21 |
| P,X | 手の平で物に触れる行動と唾液中のオキシトシン量との関連性を確認, 花王株式会社 ニュースリリース［オンライン］, 29 January 2019, [retrieval date 08 August 2019], Internet: <URL:https://www.kao.com/jp/corporate/news/2019/20190129-001/>, non-official translation ("Confirmation of the relationship between the action of touching objects with the palm and the amount of oxytocin in saliva", KAO CORP., News Release [online]) | 1-34 |

Form PCT/ISA/210 (continuation of second sheet) (January 2015)

## REFERENCES CITED IN THE DESCRIPTION

### Patent documents cited in the description

- WO 2014092087 A
- JP 2014113302 A
- JP 2015112343 A

### Non-patent literature cited in the description

- **LIEBERWIRTH ; WANG.** *CURRENT OPINION IN NEUROBIOLOGY,* 2016
- **LOKEN et al.** *NATURE NEUROSCIENCE,* 2009, vol. 12, 547-548
- **RIE HIKIMA et al.** Program of the 35th Meeting of the Japanese Society for Physiological Psychology and Psychophysiology. *Preprints,* 2017, vol. 59
- **MORHENN et al.** *Altern. Ther. Health. Med.,* 2012, vol. 18, 11-18
- **ANDARI E. et al.** *Proc Natl Acad Sci USA,* 2010, vol. 107 (9), 4389-94
- **YAMASHITA ; KITANO.** *Mol. Phylogenet. Evol.,* 2013, vol. 2, 520-528
- The standardization and analysis of hand evaluation. **SUEO KAWABATA.** The Textile Machinery Society of Japan, Hand Evaluation and Standardization Committee. 10 July 1980